# EUROPEAN PATENT APPLICATION

(11) **EP 2 957 910 A1**
(43) Date of publication of application: **23.12.2015**
(21) Application number: 14751079.6
(22) Date of filing: 12.02.2014
(51) Int. Cl.: G01N 33/53, A61K 38/30

(54) **USE OF IGF-1 AS A REAGENT FOR EARLY DIAGNOSIS AND/OR PROGNOSIS OF ALZHEIMER DISEASE**

(30) Priority: 13.02.2013 ES 201330187
(71) Applicant: Consejo Superior De Investigaciones Científicas (CSIC), 28006 Madrid (ES); Universidad Autónoma De Madrid (UAM), 28049 Madrid (ES)
(72) Inventor: TRUEBA SAIZ, Angel, 28006 Madrid (ES); TORRES ALEMAN, Ignacio, 28006 Madrid (ES); CAVADA MARTINEZ, Carmen, 28049 Madrid (ES); NUÑEZ MOLINA, Angel, 28049 Madrid (ES)
(74) Representative: Pons Ariño, Angel
(86) International application number: PCT/ES2014/070103
(87) International publication number: WO 2014/125150

(57) **Abstract**

The invention relates to the use of the insulin-like growth factor (IGF-1) as a reagent for the early diagnosis and/or prognosis of Alzheimer disease, to a kit comprising IGF-1 and a sedative, and to the use thereof in the early diagnosis and/or prognosis of Alzheimer disease.

## Description

### FIELD OF THE INVENTION

The invention relates to the use of insulin-like growth factor-1 (IGF-1) as early diagnostic and/or prognostic reagent of Alzheimer's disease, and to a kit comprising IGF-1 and a sedative and its use in the early diagnosis and/or prognosis of Alzheimer's disease. Therefore, the invention is included in the field of biomedicine and more specifically in the field of early diagnosis or prognosis of Alzheimer's disease (AD).

### STATE OF THE ART

Currently, AD is one of the main health problems in modern societies due to its high incidence. After unsuccessful years of looking for effective therapies, it has been recently been shown that the failure of the numerous therapeutic tests performed could be due to the fact that the treatments are always started in too advanced phases of the disease since the current diagnostic methods (fundamentally based on clinical scales) do not make it possible to select the patients in early phases of the pathology.

There are two fundamental lines of search for diagnostic methods, one based on advanced techniques of medical imaging and another on genomic and proteomic techniques. Both are producing many innovations but none has yet become installed in clinical practice. Furthermore, none claims yet to be an early diagnosis system. In general, these methods are based on the more accepted hypothesis as origin of the disease, the "amyloid cascade" (Selkoe, D.J., 2001, Neuron 32, 177-180). Therefore the diagnostic methods are especially, though not exclusively, focussed on detecting biomarkers related to this same hypothetical pathogenic mechanism.

Thus, in the diagnosis of AD the use has been disclosed of a monoclonal antibody directed at the beta amyloid peptide (ES2259270B1), the analysis of polymorphisms in the il-10 gene (EP1509621 B1), the measurement of b-amyloid peptide and Tau protein concentration (EP0792458B1) or the analysis of the phosphorylation of a mitogen-activated kinase (EP1385531 B1). In 2012, the FDA approved the use of the Florbetapir compound, capable of binding to b-amyloid proteins and being detected by positron emission tomography (PET), as reagent for the diagnosis of AD.

Alternatively, different hypotheses on the origin of the disease have recently been arising or resurging. One of them upholds that AD obeys a metabolic imbalance where insulin would be involved (Blum-Degen, D. et al. 1995, J. Neural Transm. Suppl 46, 139-147) and possibly one of its related factors, insulin-like growth factor-1, also known as somatomedin C, or IGF-1 (insulin-like growth factor-1) (Carro, E. & Torres-Alemán, I. 2004, J Pharmacol. 490, 127-133).

Therefore, in the state of the art, there is the need to develop early diagnosis methods of AD alternative to those already in existence.

### DETAILED DESCRIPTION OF THE INVENTION

The present invention provides the use of IGF-1 to detect the underlying pathology in patients with AD, in even preclinical stages. The results obtained in mice and in monkeys, wherein they were administered a systemic injection of IGF-1 to determine its effect on the electroencephalogram (EEG), make it possible to conclude that in preclinical phases of AD, the EEG signal is altered in response to the administration of IGF-1, so that this process is useful for the early diagnosis and/or the prognosis of AD in human patients.

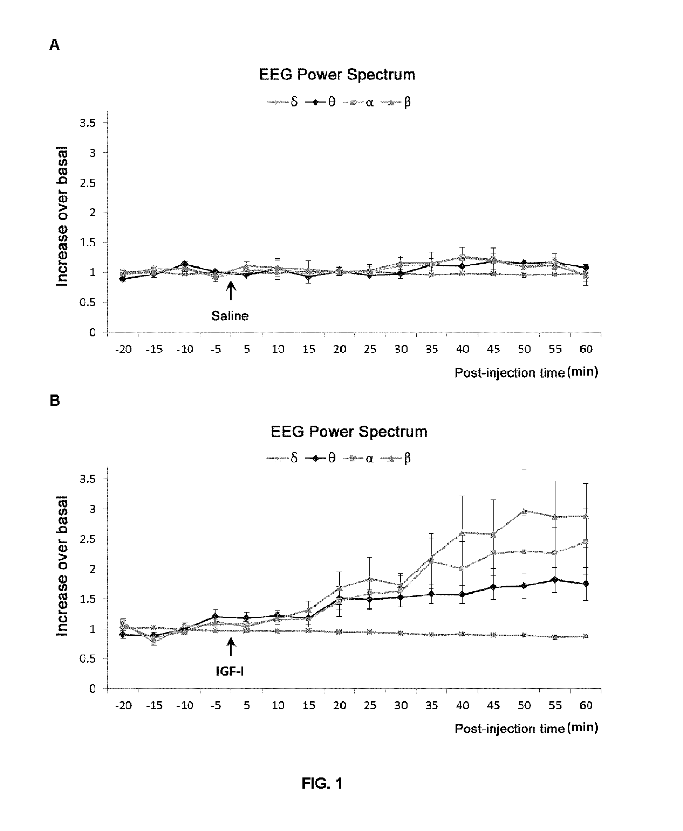

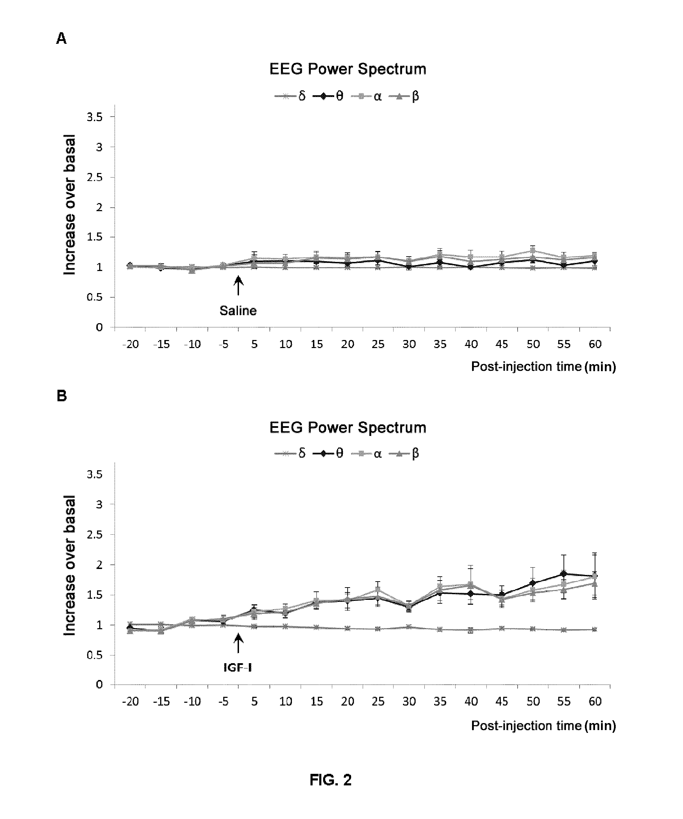

The present invention is based on what the inventors have observed that whilst IGF-1 administered to healthy animals (in conditions of sedation) modifies the α, β, δ, and θ EEG frequency bands recorded, in animals with AD these changes are not observed. More specifically, and as is shown in Figure 1, whilst the injection of saline solution does not alter the EEG frequencies recorded (α, β, δ, and θ), in healthy control mice IGF-1 causes a sustained and significant increase of 3 of them (α, β, and θ) and a small drop in the δ waves. The same occurs in healthy control monkeys (Figure 2), indicating that the effect of IGF-1 is also present in primates.

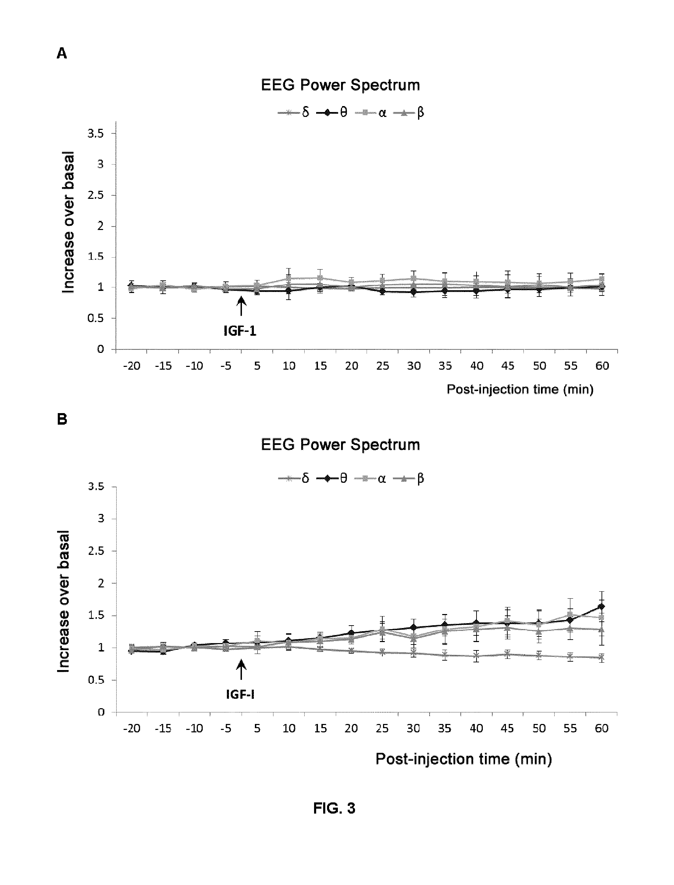

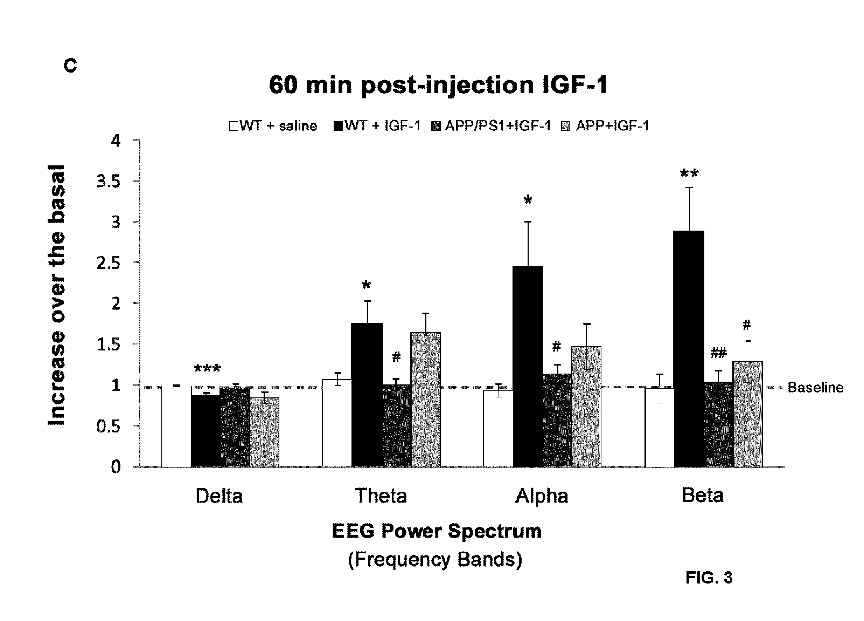

In contrast, if IGF-1 is administered to APP/PS1 mice of the same age (3-4 months) which model Alzheimer's-type amyloidosis and which are in the initial phases of the pathology this increase in the EEG frequency bands (α, β, and θ) is not observed, indicating that IGF-1 stops acting in the brain with Alzheimer-type pathology (Figure 3).

Next, young APP mice were used (3-5 months) which correspond to another model of genetically modified mouse which develops the Alzheimer-type pathology much slower, so that up to 9-10 months they are cognitively intact and do not have amyloid plaques in the brain. However, these APP mice without pathology also show an attenuated response to IGF-1, in this case in the α and β frequencies. This indicates that the loss of sensitivity to IGF-1 appears before the clinical symptoms of the disease (Figure 3).

Therefore, the type of response to IGF-1 makes it possible to quickly discriminate between healthy and sick individuals and, more significantly, from those that will suffer from the disease in the future but at the time of the test are asymptomatic, distinguishing healthy animals from those that are going to develop the AD pathology due to the attenuation of the α and β bands of the EEG. Furthermore, if band θ is also attenuated, that means that the pathology is already present.

Therefore, a first aspect of the present invention relates to the use of insulin-like growth factor-1 (IGF-1) as early diagnostic and/or prognostic reagent of AD.

In the present invention "insulin-like growth factor-1" (IGF-1) or somatomedin C, or IGF-1 is understood to be the protein which in humans is coded by gene *IGF-1.* The IGF-1 protein is a hormone similar in molecular structure to insulin, the molecular weight of which is 7,649 Da, which consists of 70 amino acids in a single chain with three intramolecular disulphide bridges. Its main action is mediated by the bond to its specific receptor, the insulin-like growth factor-1 receptor (IGF1R). IGF-1 is one of the most potent natural activators of PKB (Protein Kinase B) signal transduction, a growth and cell proliferation stimulator, and a potent inhibitor of programmed cell death.

In the present invention, "diagnostic reagent" is understood as that compound which serves to diagnose a disease, in particular, AD, and "prognostic reagent" refers to that compound which serves to prognosticate a disease, in particular, AD. In the present invention, said reagent is IGF-1 which, when it is administered to an individual, is capable of modifying the EEG bands differentially depending on whether said individual suffers from AD or not. This reagent is, therefore, a diagnostic and/or prognostic reagent which makes it possible to determine if an individual suffers from AD or not, or to determine if an individual will develop AD or not.

The term "early diagnosis of AD" refers to the process comprising determining whether an individual suffers from AD or not before the early symptoms of the disease appear, including, without being limited to, confusion, alterations in the short-term memory, attention and spatial orientation problems, changes in personality, language difficulties and unexplainable mood changes.

The term "early prognosis of AD" refers to the process whereby a prediction is established of the events which will occur in the development or course of AD, including increase in the seriousness of the disease in subjects in the initial stages thereof, i.e. before the appearance of clinical symptoms of AD.

The IGF-1 protein can be administered to an individual by any of the routes of administration known by the person skilled in the art. Examples of routes of administration include, but are not limited to, peritoneal, cutaneous and parenteral. However, a preferred route of administration is parenteral, i.e. the subcutaneous injection of IGF-1.

Likewise, the person skilled in the art understands that IGF-1 can be administered forming part of a pharmaceutical composition that will comprise IGF-1 and a pharmaceutically acceptable vehicle. The pharmaceutical composition may be in any pharmaceutical form of administration which is considered suitable for the route of administration chosen, for example, by systemic, oral, parenteral or topical route, for which purpose it will include the necessary pharmaceutically acceptable excipients for the formulation of the desired form of administration.

Illustrative, but non-limiting examples of pharmaceutical forms of administration by oral route include tablets, capsules, granules, solutions, suspensions, etc., which may contain the appropriate conventional vehicles, such as binders, diluents, disintegrants, lubricants, wetting agents, etc., and may be prepared by conventional methods known by persons skilled in the art. The pharmaceutical compositions may also be adapted for their parenteral administration, in the form of, for example, solutions, suspensions, in the form of appropriate dosing; in this case, said pharmaceutical composition shall include the suitable pharmaceutically acceptable vehicles, such as buffers, surfactants, etc., and may be prepared by conventional methods known by the persons skilled in the art. In any case, the pharmaceutically acceptable vehicles shall be chosen in accordance with the pharmaceutical form of administration selected. A review of the different pharmaceutical forms of administration of drugs and of the pharmaceutically acceptable vehicles necessary for their obtainment as well as of their production methods can be found, for example, in "Tratado de Farmacia Galénica", C. Faulí i Trillo, 1993, Luzán 5, S.A Editions, Madrid; and in Remington's Pharmaceutical Sciences (AR. Gennaro, Ed.), 20th edition, Williams & Wilkins PA, USA (2000).

The expression "pharmaceutically acceptable vehicle", as used here, refers to a vehicle which must be approved by a regulatory agency of the federal government or a state government or listed in the United States Pharmacopoeia or the European Pharmacopoeia, or another pharmacopoeia generally recognized for its use in animals and more specifically in humans. The term "vehicle" refers to a diluent, coadjuvant, excipient or carrier whereby the active principle must be administered (i.e. IGF-1); obviously, said vehicle must be compatible with said products.

As understood by the person skilled in the art, the concentration of IGF-1 in the pharmaceutical composition shall be in an effective concentration, i.e. in a concentration so that it can exercise its activity, such as alter the pattern of EEG bands when it is administered to a subject. The effective quantity of IGF-1 may vary within a wide range, and in general, will vary depending on particular circumstances of application, of the subject that is going to be treated, their age, their condition, the form of administration chosen, the route and frequency of administration and considerations of this type. Thus, in a particular embodiment, the concentration of IGF-1 is between 50 and 300 µg/kg, in particular, between 100 and 250 µg/kg. In an even more particular embodiment, the concentration of IGF-1 is 150 µg/kg.

The term "subject", as used here, refers to a member of a species of a mammal animal and includes, but is not limited to, domestic animals, primates and humans; preferably said subject is a male or female human being of any age or race.

"Alzheimer's disease" or "AD" is a neurodegenerative disorder characterized by the progressive deterioration of mental processes and the function of the memory, communication problems, changes of personality, erratic behaviour, dependency and the loss of control over body functions.

On the other hand, as explained at the beginning of the present description, the invention is based on the fact that the administration of IGF-1 to healthy animals modifies the α, β, δ, and θ EEG frequency bands recorded, whilst in animals with AD these changes are not observed. To be able to observe said changes in the frequency bands it is necessary that the subject is calm or in a stable basal state. For the person skilled in the art it is routine practice to judge when this point of the process has been reached.

"Stable basal state" refers to the time when in the patient, being in sensorial and mental rest (with or without sedation), has an electroencephalographic activity such that it empirically has the minimum variation in accordance with time i.e. its composition of frequency bands spontaneously does not vary or does so minimally. If that was not possible, then there is the possibility of administering a sedative with the purpose of achieving said stable basal state. Thus, in a particular embodiment, IGF-1 is sequentially used with a sedative.

In the present invention "sequential use" is understood as the administration of a first reagent and, then, the administration of a second reagent, being able to exercise their effects simultaneously over time. In the context of the present invention, the first reagent is a sedative and the second reagent is IGF-1. The time of administration between both reagents may vary, but the second reagent (IGF-1) shall not be administered until the first reagent (sedative) exercises its effect on the individual and a stable basal state is obtained. Both reagents shall be administered to the patient by systemic route, preferably, through the intravenous route (although subcutaneous is equally valid) being acceptable any formulation of the reagents aimed at said route of administration, so that those typically used by the person skilled in the art can be used.

"Sedative" refers to an active principle which, when administered causes in the subject, among other effects, calm, relaxation, reduction of anxiety or sleepiness to thus facilitate that the subject has a "stable basal state" and thus (i) minimize the differences with the experimental paradigm and (ii) obtain greater homogeneity in the results of the diagnostic test. Any sedative known by any person skilled in the art for said purpose can be used and which is preferably a compound of the family of benzodiazepines, for example, but without being limited to, triazolam, oxazolam, estazolam, diazepam, lorazepam, lormetazepam, bentazepam, flurazepam, flunitrazepam, clonazepam, clorazepate dipotassium and chlordiazepoxide.

Thus, in a particular embodiment, the sedative belongs to the group of benzodiazepines. Preferably, the sedative is selected from the group consisting of triazolam, oxazolam, estazolam, diazepam, lorazepam, lormetazepam, bentazepam, flurazepam, flunitrazepam, clonazepam, clorazepate dipotassium and chlordiazepoxide.

In the present invention, the application of the sedative has to be such that it causes a slight or minimum sedation, also called ansiolysis, without the need for the presence of an anaesthetist. Ansiolysis is a state induced by drugs where the patient responds normally to verbal commands, breathing and the cardiovascular function remain unaltered; however, the cognitive function and motor coordination may be attenuated. Likewise, it is also possible that with the sedation the patient passes to a moderate sedation level known in clinical practice as "conscious sedation". Conscious sedation is a medically controlled state of decrease in conscience, but conserving the protective reflects. Thus it is preserved the patient's capacity to maintain their breathing independently and continuously, in addition to allowing response to physical stimuli and orders.

The light sedation or conscious sedation dose of the patient in the present invention is governed according to the sedation process guides during diagnostic tests, existing in all the hospitals. Therefore, the administration of the light sedation or conscious sedation dose during diagnostic tests is for the person skilled in the art a routine practice.

On the other hand, the person skilled in the art understands that the administration of IGF-1 at high concentrations may cause hypoglycaemia in the individual, which may alter the pattern of EEG frequency bands. For this reason, optionally, before measuring said band pattern, the blood sugar levels may be measured before and after the administration of IGF-1 and thus control glycaemia in the individual. The person skilled in the art knows how to evaluate when the IGF-1 concentration is sufficiently high to be able to cause hypoglycaemia in the individual, and in what conditions IGF-1 may have this effect (for example, if the individual has not eaten). Therefore, the person skilled in the art knows when it is recommendable to control glycaemia. Any conventional technique to measure blood sugar levels may be used in the present invention.

In another aspect, the present invention is related to a kit, hereinafter "kit of the invention", comprising insulin-like growth factor-1 (IGF-1) and a sedative.

The terms "insulin-like growth factor-1" (IGF-1) and "sedative" have been defined or explained previously in the present description.

In the present invention, a kit is understood as a product which contains the different active principles or compounds of the invention (i.e. IGF-1 and a sedative) forming a unit which allows its transport, storage and its sequential administration. The kit of the invention may contain in this way one or more suspensions, tablets, capsules, inhalers, syringes, patches and similar, which contain the active principles of the invention and which may be prepared in an individual dose or as multiple doses. The kit may additionally contain a suitable support to resuspend the active compounds such as an aqueous medium, saline solution, etc. Other components which may be present in the kit is a container which makes it possible to maintain the active principles within certain limits. The suitable materials to prepare said containers include glass, plastic, polyethylene, polypropylene, polycarbonate and similar, bottles, vials, paper, sachets and similar.

The kit of the invention may additionally contain instructions for the sequential or separate administration of the different active principles present in the kit. Therefore, in a form of particular embodiment, the kit of the invention also comprises instructions for the simultaneous, sequential or separate administration of the different components and/or to carry out the early diagnosis and/or prognosis of AD. Said instructions may be found in the form of printed material or in the form of electronic format which may store the instructions such that they can be read by a subject such as electronic storage media (magnetic disks, tapes and similar), optical media (CD-ROM, DVD) and similar. The media may additionally or alternatively contain websites in the internet providing said instructions.

The concentration of IGF-1 in the kit of the invention may vary in a wide range of concentrations. However, in a particular embodiment, the concentration of IGF-1 is between 50 and 300 µg/kg, in particular, between 100 and 250 µg/kg. Preferably, the concentration of IGF-1 of the kit of the invention is of 150 µg/kg.

The kit of the invention may comprise any sedative known by the person skilled in the art to achieve a stable basal state in an individual. In a particular embodiment of the kit of the invention, the sedative belongs to the group of benzodiazepines. Preferably, the sedative is selected from the group consisting of triazolam, oxazolam, estazolam, diazepam, lorazepam, lormetazepam, bentazepam, flurazepam, flunitrazepam, clonazepam, clorazepate dipotassium and chlordiazepoxide.

These particular embodiments have been cited, described and explained in previous inventive aspects and, in consequence, it is not necessary to make further comments in this respect.

As has been explained above, the administration of IGF-1 at high concentrations may cause hypoglycaemia in the individual, which may alter the frequency band pattern. Thus, in another particular embodiment, the kit of the invention comprises compounds to determine the blood glucose. In an even more preferred embodiment, the compounds to determine the blood glucose are reactive strips.

"Reactive strips" in the present invention are understood as strips which are used to determine the blood glucose concentration based on the functioning of the glucose oxidase enzyme. The strips impregnated with the glucose oxidase enzyme and ferrocene have a chip and are coupled to a reader. The glucose oxidase enzyme contained in the blood sample and transfers electrons to ferrocene, which also transfers these electrons to the chip, which generates current intensity. The reader, called glucometer translates the current intensity value into blood glucose concentration.

Finally, in another aspect, the present invention relates to the use of the kit of the invention for the early diagnosis and/or prognosis of AD. The meaning of the terms used in the present inventive aspect as well as its particular embodiments have been explained in previous inventive aspects.

Furthermore, within the context of the present invention it also includes a method for the early diagnosis of AD of a subject comprising:
(a) Measuring the α, β, δ, and θ EEG frequency bands of said subject in a basal state,
(b) Administering to said subject a therapeutically effective quantity of IGF-1, and
(c) Again measuring the α, β, δ, and θ EEG frequency bands of said individual,
wherein if the α, β and θ band frequency pattern obtained before the administration of IGF-1 does not significantly vary with respect to the pattern of bands obtained after the administration of IGF-1, then the subject suffers from AD.

Likewise, the present invention also includes a method for the early prognosis of AD of a subject comprising:
(a) Measuring the α, β, δ, and θ EEG frequency bands of said subject in a basal state,
(b) Administering to said subject a therapeutically effective quantity of IGF-1, and
(c) Again measuring the α, β, δ, and θ EEG frequency bands of said individual,
wherein if the pattern of α and β bands obtained before the administration of IGF-1 does not significantly vary with respect to the pattern of bands obtained after the administration of IGF-1, then the subject is going to develop AD.

Optionally, before measuring the α, β, δ, and θ EEG frequency bands in said subject, the methods of the invention may comprise another stage aimed at measuring the blood sugar levels in the individual, both before and after the administration of IGF-1 with the aim of controlling glycaemia, since as previously explained, alterations therein may modify the EEG pattern and IGF-1 also has hypoglycaemia-causing potential at high doses.

The meaning of the terms used in the method of diagnosis mentioned here has already been explained throughout the present description. Likewise, particular embodiments of said method of diagnosis include, but are not limited to, the particular embodiments described for the inventive aspects of the present invention.

Throughout the description and the claims the word "comprises" and its variants are not intended to exclude other technical characteristics, additives, components or steps. For persons skilled in the art, other objects, advantages and characteristics of the invention will be inferred in part from the description and in part from the practice of the invention. The following examples are provided by way of illustration, and are not intended to limit the present invention.

### BRIEF DESCRIPTION OF THE FIGURES

**Figure 1****.- Effect of IGF-1 on the EEG profile of healthy mice.** A, Response to systemic administration of saline solution (n= 9). B, Response to IGF-1 (1 mg/kg). The 4 frequency bands analysed (δ, θ, α, β) were significantly modified by IGF-1 (n=14) as revealed by the one-way ANOVA test (δ: F=14.20; p<0.001 / θ: F=37.53; p<0.001 / α: F=18.50; p<0.001 / β: F=22,41; p<0.001). It isshown the mean response of control mice.
**Figure 2****.- Effect of IGF-1 on the EEG profile of healthy adult monkeys.** A, Response to systemic administration of saline solution (n=7). B, Response to IGF-1 (100 µg/kg). The 4 frequency bands analysed (δ, θ, α, β) were significantly modified by IGF-1 (n= 5) as revealed by the two-way ANOVA test of repeated measurements (δ: F=10.18; p<0.01 / θ: F=13.91; p<0.01 / α: F=6.235; p<0.05 / β: F=8,48; p<0.05). It is shown the mean response.
**Figure 3****.- Stimulation by IGF-1 of the EEG waves is altered in mice with Alzheimer-type amyloidosis before symptoms of the disease appear.** The response to IGF-1 after 1 hour of its systemic administration generated significant increases in the frequency bands: α, β, and θ, and a small decrease, also significant, in the δ band in healthy control mice (wt). A, This response pattern is absent in APP/PS1 mice (δ: F=14.20; p<0.001 / θ: F=37.53; p<0.001 / α: F=18.50; p<0.001 / β: F=22.41; p<0.001) which already suffered from the symptoms of the disease whilst in B, APP mice which did not have still any symptoms were also significantly attenuated in the θ, α and β frequency bands (θ: F=37.53; p<0.01 / α: F=18.50; p<0.001 / β: F=22.41; p<0.001). C, After 60 minutes of treatment (saline or IGF-1); Student T vs WT + Saline (*p<0.05, **p<0.01, *** p<0.001) or vs WT + IGF-1 (^{#}p<0.05, ^{##}p<0.01, ^{###}p<0.001)

### EXAMPLES

### Materials and Methods:

### Offline analysis and Statistics

The Spike 2 program was used to calculate the EEG power spectrum using a resolution of 0.2 Hz in recording intervals of 5 minutes and the following frequency bands were analysed: Delta band (0.3-4 Hz), Theta band (4-8 Hz), Alpha band (8-12 Hz) and Beta band (12-30 Hz). The contribution percentage of each frequency band to the EEG overall was calculated and said value was normalized compared with the baseline (calculated as the mean EEG during the control period). Thus the values graphically represented in the figures in accordance with time, considering the injection of IGF-1 or saline solution as t = 0 minutes.

For statistical analysis, the GraphPrism program was used and the following statistical analyses were performed: Student T and two-way ANOVA. Statistically significant differences were considered those with p<0.05.

All data are represented as mean ± SEM (standard error of the mean).

### Example 1.- Initial experiments with mice.

All experiments were carried out in accordance with R.D. 1201/2005 on protection of animals used for experimentation and other scientific purposes.

It also had the approval of the Research Ethics Committee of the Universidad Autónoma de Madrid (UAM). The animals were housed until the time of the experiment in groups of a minimum of 2 mice, in constant conditions of humidity (55 ± 10%) and temperature (22 ± 2°C) and under a 12 hour cycle of light/dark with food and drink *ad libitum.*

For the experimental processes, mice of 3-4 months of age between 25-30 g of weight and of both sexes were used. The animals of the strain wild C57BL6/J or *wild type* (WT) were supplied by the Veterinary Office of the Faculty of Medicine of the UAM (Madrid, Spain). The APP/PS1 genetically modified animal model was provided by the NIH (U.S.A.): they are mice of the C57BL6/J strain which express two mutated human genes (APPswe and PSEN1dE9) causing familial Alzheimer's disease under the control of the murine promotor of prion protein (PrP). Via back-crossing with WT animals we obtained another genetically modified animal model of the disease which only carried the APPswe (APP) transgene and which has a disease of later appearance and slower progression.

At the end of the experiment each animal was sacrificed by anaesthetic overdose or intracardiac perfusion.

During the processes, isofluorane was used both in the induction and maintenance phase. For the first, a mixture of 2-3% of the anaesthetic in O₂ (0.5-1 L/minute) was used, the mouse was placed in a stereotaxic apparatus, an incision was made along the midline and at the height of the primary somatosensory cortex (IF) a craniotomy was performed with the aid of a 0.5 mm diameter bit. There, on the dura mater, a tungsten macroelectrode (0.5 MOhm resistance) (World Precision Instruments, WPI, Sarasota, FL, USA) was placed in order to record the electrical activity of the EEG. During the maintenance phase, the anaesthesia was reduced to 0.5-1.5%, in accordance with each animal so that both the absence of podal and parebral reflexes were maintained, and a stable EEG register with slow waves of large amplitude.

The protocol followed was as follows: the basal activity of the EEG was recorded during 20 minutes (control period), 25 µg of IGF-1 was injected in the peritoneum in a final volume of 100 µl of physiological solution or just the vehicle in the control registers. Next, the EEG was recorded during 1 additional hour. The electrical activity registered was filtered between 0.3 and 100 Hz, it was amplified (P15, Grass & Ampli 4G, Cibertec) and stored in a PC by analogue-digital conversion card (1401, Cambridge Electronic Design, Cambridge, UK), at a sampling frequency of 512 Hz, for its offline analysis with the Spike 2 program (Cambridge Electronic Design, Cambridge, UK).

The results are shown in the Figure 1. The control mice responded to the IGF-1 with increases in the θ, α, and β frequencies and decreases in the δ band. After one hour, the statistical differences were analysed, all of them being significant (Figure 3). When the same experiments were performed with APP/PS1 mice, it is observed that there is no type of response to the injection of IGF-1, with the profile of the evolution of the EEG over time being very similar to that of the control group injected with saline solution. In the case of the APP mice, it is observed that there is a response to IGF-1 but it is attenuated i.e. the activation of the EEG is of less intensity than in the case of the *wild type* injected with IGF-1.

### Diagnostic algorithm

To analyse the date, we created a subroutine in Matlab Programming language. We assign each column of values to an independent vector: one of voltage and another of time. We analysed the data in 5 minute intervals and performed the power spectrum for each interval with a function which uses the multitaper method described by Percival et al., 1993, Cambridge University Press which uses linear and non-linear combinations of periodgrams modified to obtain the power spectral density (PSD).

For the use of the function we will enter the values: signal voltage value, time of bandwidth predetermined by the function, sampling size to perform Fourier transform (nfft=512) and the sampling frequency. The function gives us two vectors: power (V^2) and frequency (Hz).

Once the PSD has been obtained, we separate the data into different frequency bands with the following criteria: delta frequency band from 0.3 to 4 Hz, theta frequency band from 4 to 8Hz, alpha frequency band from 8 to 12 Hz and beta frequency band from 12 to 30 Hz. We add together all signal points comprising each frequency interval, obtaining with this two different values: the power percentage of each frequency and the mean power for each frequency throughout the signal. With these two values we can obtain the increase on the basal ("fold increase") of the power for each frequency band throughout the 5-minute recording time.

In this way, in a control case (wild type): the delta frequency does not vary more than 20% for 80% of cases; the theta frequency varies more than 25% for 80% of cases; the alpha frequency varies more than 38% for 80% of cases; and the beta frequency varies more than 45% in 100% of cases (Figure 3).

### Example 2.- Experiments with non-human primates (NHP).

To perform the EEG recordings in non-human primates, nine healthy adult macaque monkeys were used (two *Macaca fascicularis* and seven *Macaca mulata,* from 12 to 18 years of age and weight from 3.6 to 11.7 kg. All the individuals were male with the exception of a *Macaca fascicularis* female. The monkeys were supplied by R.C. Hartelust BV (Tilburg, Netherlands) and remain housed in the Veterinary Office of the Faculty of Medicine of the UAM (registration no. EX 021-U) with controlled breathing (16 L/minute), temperature (22 ± 4°C), humidity (55-65%) and light/dark cycle conditions (12 hours). They were fed with commercial food and had free access to water.

The animals were supervised by the Director of the Veterinary Office of the UAM fulfilling the Spanish and European directives (R.D. 1201/2005, Act 32/2007 and European Directives 86/609/EEC and 2003/65/EC). It also had the approval of the Research Ethics Committee of the UAM.

Eight monkeys were used as control injected with saline solution and five were injected with IGF-1.

The animals were sedated with ketamine (5-10 mg/kg intramuscular) and anaesthetized with isofluorane (2-4% during induction; 1-1.5% during maintenance) mixed with O₂ (2 L/minute during induction; 1.5 L/minute during maintenance). A sufficient time was waited (15-30 minutes) to ensure the clearance of ketamine and the stability of the EEG recording before starting with the definitive recording. To avoid artefacts derived from IGF-1-induced hypoglycaemia, the glucose blood levels were controlled and maintained in normoglycaemia ranges *(M. fascicularis:* 50-70 mg/dl; *M. mulata:* 40-60 mg/dl) by systemic infusion of salt-sugar solution)(3.3%) (Grifols, Spain) through a catheter placed in the small saphenous vein.

To record the EEG, three surface electrodes (World Precision Instruments) were used placed in F_{pz}, C_{z} and O_{z} according to the 10-20 System for EEG Recording. This is a standardized international system which uses the *nasion* and the *inion* as reference points to place the electrodes in different percentage distances to them (F_{pz} at 10% *nasion,* C_{z} equidistant to both and O_{z} at 10% *inion*) each one with their corresponding reference electrodes and ground electrodes. To improve the signal conductivity, an electrically conductive gel was used between the surface of the scalp and the electrode. From this time, the experimental protocol was identical to that of the mouse with the only exception that both the saline solution and IGF-1 (100 µg/kg diluted at 1 mg/mL in saline solution) were injected as an intravenous bolus through the venous route.

The signal processing was similar with the exception that it was included in three P15 amplifiers, Grass and then it was filtered in an Ampli 4G filter/amplifier, Cibertec 4-channel. It was also digitalized to 512 Hz using the CED 1401 card and analysed offline as previously described.

The results are shown in Figure 2. The monkeys that received IGF-1 responded identically to the mice, with increases in the θ, α, and β bands and a slight decrease in the δ band.

## Claims

1. Use of insulin-like growth factor-1 (IGF-1) as early diagnostic and/or prognostic reagent of Alzheimer's disease.

2. Use according to claim 1, wherein the concentration of IGF-1 is between 50 and 300 µg/kg, in particular, between 100 and 250 µg/kg.

3. Use according to claim 2, wherein the concentration of IGF-1 is 150 µg/kg.

4. Use according to any of claims 1 to 3, wherein IGF-1 is sequentially used with a sedative.

5. Use according to claim 4, wherein the sedative belongs to the group of benzodiazepines.

6. Use according to claim 5, where the sedative is selected from the group consisting of triazolam, oxazolam, estazolam, diazepam, lorazepam, lormetazepam, bentazepam, flurazepam, flunitrazepam, clonazepam clorazepate dipotassium and chlordiazepoxide.

7. Kit comprising insulin-like growth factor-1 (IGF-1) and a sedative.

8. Kit according to claim 7, wherein the concentration of IGF-1 is between 50 and 300 µg/kg, in particular, between 100 and 250 µg/kg.

9. Kit according to claim 8, wherein the concentration of IGF-1 is 150 µg/kg.

10. Kit according to any of claims 7 to 9, wherein the sedative belongs to the group of benzodiazepines.

11. Kit according to claim 10, wherein the sedative is selected from the group consisting of triazolam, oxazolam, estazolam, diazepam, lorazepam, lormetazepam, bentazepam, flurazepam, flunitrazepam, clonazepam clorazepate dipotassium and chlordiazepoxide.

12. Kit according to any of claims 7 to 11, comprising compounds for the determination of the blood glucose.

13. Kit according to claim 12, where the compounds for the determination of the blood glucose are reactive strips.

14. Use of a kit according to any of claims 7 to 13, for the early diagnosis and/or prognosis of Alzheimer's disease.
